# EUROPEAN PATENT APPLICATION

(11) **EP 4 404 203 A1**
(43) Date of publication of application: **24.07.2024**
(21) Application number: 23152363.0
(22) Date of filing: 19.01.2023
(51) Int. Cl.: G16B 50/30

(54) **COMPUTER-IMPLEMENTED METHOD FOR ANALYZING SOURCE DATA DESCRIBING A GENOME SEQUENCE**

(71) Applicant: Siemens Healthcare Diagnostics Products GmbH, 35041 Marburg (DE)
(72) Inventor: Klatt, Torbjörn, 50374 Erftstadt (DE)
(74) Representative: Siemens Healthineers Patent Attorneys

(57) **Abstract**

The invention concerns a computer-implemented method for analyzing nucleic acid sequences data, i.e. source data describing a genome sequence, in a processing device (1) having at least one processor (4) and at least one local memory (5) used by the at least one processor (4), wherein the source data is stored in at least one source file (2, 2a) in a storage (3). The invention further concerns a processing device (1) and a processing system (13).

## Description

The invention concerns a computer-implemented method for analyzing source data describing a genome sequence in a processing device having at least one processor and at least one local memory used by the at least one processor, wherein the source data is stored in at least one source file in a storage. The invention further concerns a processing device and a processing system.

The analysis and evaluation of genomic and transcriptomic data has become an often-used tool in medical research and clinical diagnosis. Here, in the following, genomic and transcriptomic data as input data to an analysis will be commonly named source data describing a genome sequence. For example, such source data may be analyzed in analysis processes, which yield result information which may, for example, be further used in diagnosis to be performed by a physician. With increasing efficiency and significance of such genomic and transcriptomic tests, the number of source data sets to be evaluated grows, as does the time between sample-taking, measurement and availability of the results. The time span between taking of the sample and receiving the results of a corresponding analysis process is generally also called time-to-solution. A general objective is to reduce this time-to-solution, in particular regarding the increasing digitalization and globalization, in particular regarding the prevalence of cloud-based IT systems, and the resulting importance of resource and cost efficiency of the genomic and transcriptomic data analysis.

Here, a major challenge regarding bioinformatic problemsolving in the analysis of source data describing genome sequences is the amount of data for each case, that is, each single sample. For example, data regarding a whole genome sequence (WGS) may have a size of one TB, whole exome data a size of 400 to 600 GB and even targeted panels may still result in file sizes of up to 80 GB. These source data must be read from long-time storage, for example hard disks and/or data lakes, via a communication link into the local random access memory for each step of the analysis process. The bandwidth of communication links between processing arrangements, comprising at least one processor and at least one associated local memory, to such storages, are by several orders of magnitude smaller than the bandwidth between the local memory and process memory, in particular the cache of the processor. Hence, time for processing is lost when the source data to be analyzed are awaited.

Generally speaking, processing devices for source data describing genome sequences may comprise at least one processor, which may comprise one or multiple cores and/or use hyper-threading. The at least one processor comprises a cache, which may, for example, have a size of 96 KB within a core, 1.25 MB next to core, and 8 MB shared across all cores. A local random access memory (RAM) is associated with and used by the at least one processor, which may be a CPU. The local memory is usually volatile and may have a size of several GB up to a few TB. The storage may be a hard disk, in particular an SDD and/or an HDD, of the processing device, which is non-volatile and allows to store source data over long time periods. In addition or alternatively, a remote storage, in particular a network storage, may be used, for example a cloud storage.

Examples for such source data comprise raw sequencing data, for example in the FASTQ format, base sequences mapped to a reference, for example in the BAM or CRAM format, or the sequences of a reference itself, for example in the NIB format. Such source data describing a genome sequence is usually stored in source files, which may also be called genomic files.

As already explained, such source data files have a considerable size, such that, in relation to the required processing, it takes a very long time to open them, that is, transfer them from the long-time storage into the local memory. This, however, has to be repeated for each step of the analysis process requiring information from the source data. Hence, finding single required information in these large genomic files takes a long time due to their size and the limited bandwidth between storage and local memory as well as between processor and local memory.

Known bioinformatic approaches are based on the concept of storage-optimized algorithms. Here, the amount of source data to be loaded and/or the time points, at which these source data are to be loaded, are optimally structured in the algorithm itself, in particular to bridge waiting time for source data from the storage. Often, so-called index files are employed, which accelerate locating information in source files. Such index files, however, come with the disadvantage that they have been generated separately and elaborately for each search scenario.

Other approaches try to optimize time-to-solution by using dedicated hardware components, for example graphics processing units (GPUs) or field programmable gate arrays (FPGAs) to accelerate partial algorithms/components of the analysis software. However, these approaches are also limited by the bandwidth between long-time storage, local memory and processor cache of the hardware components.

In an article by Klus, P. et al. "BarraCUDA - a fast short read sequence aligner using graphics processing units", BMC Research Notes 5 (2012), page 27, it was proposed to port the most computational-intensive alignment component of the sequence aligner BWA to a graphics processing unit to take advantage of the massive parallelism.

In an article by Zhang, Y. et al., "Efficient Memory Access-Aware BWA-SMEM Seeding Accelerator for Genome Sequencing", 2021 IEEE 23rd International Conference on High Performance Computing & Communications; 7th International Conference on Data Science & Systems; 19th International Conference on Smart City; 7th International Conference on Dependability in Sensor, Cloud & Big Data Systems & Application (HPCC/DSS/SmartCity/DependSys), 2021, pages 611 - 617, FPGA Acceleration for genome sequencing algorithms is discussed, wherein, in particular, the seeding step (BWA-SMEM) is analyzed, wherein an Efficient Memory Access-Aware BWA-SMEM Seeding Accelerator is proposed, aiming at common FPGA platforms with multiple memory channels.

In an article by Wu, L. et al., "FPGA Accelerated INDEL Realignment in the Cloud", 2019 IEEE International Symposium on High Performance Computer Architecture (HPCA), 2019, pages 277 - 290, hardware accelerators in the cloud to improve the runtime and throughput of immense-scale genomic data analysis are explored. Here, the hardware accelerator for INDEL realignment and a hardware-software framework elaborating FPGAs-as-a-service in the cloud is described.

However, these approaches suffer from several limitations and disadvantages. Regarding, for example, GPUs, they are very energy hungry and very expensive and provide their best performances for their designed use cases, namely mathematical operations like matrix multiplication on a huge set of data in parallel. However, often, different processing is required in genome analysis. Furthermore, additional latencies can be introduced when using a separate PCI card. Regarding FPGAs, which are very fast and energy efficient, however, switching algorithms requires re-uploading another firmware, which may take up to several minutes. Furthermore, the hardware is specialized and, in the case of PCI cards, further latencies may be introduced.

It is an object of the current invention to reduce time-to-solution for analysis processes of source data describing genome sequences.

This object is achieved by providing a computer-implemented method according to claim 1, a processing device according to claim 10 and a processing system according to claim 15. Advantageous embodiments are described in the dependent claims.

In a computer-implemented method as initially described, according to the invention, a programmable local memory, which is configured to execute at least one software means, and a selector software, which is at least partly executed in the programmable local memory, are used, wherein
- at least one required source file for a current analysis process is determined,
- the at least one required source file is loaded by the selector software from the storage into the programmable local memory,
- the selector software provides at least one selector interface to at least one analysis software component, which is executed on at least one of the at least one processor, of the current analysis process,
- wherein, if a source data request from at least one of the at least one analysis software component is received via the selector interface, requested source data is selected from the at least one required source file in the programmable local memory by at least one selector component of the selector software according to the source data request and provided via the selector interface to the requesting analysis software component.

It is hence proposed to use a dedicated combination of a hardware component and a software component, namely a programmable local, for example random access or content-addressable, memory (shortly programmable local memory) and the selector software, respectively. A programmable memory, which may also be called computable memory, has in principle already been proposed in the state of the art for other applications, and multiple approaches for implementing such a programmable memory, wherein software means programmed into the programmable memory are executed by the memory itself, have already been proposed. Generally speaking, the concept of "programmable memory" adds a programmable unit, which is conceptually similar to FPGAs, to local, for example random access or content-addressable, memory. In this manner, the idea of "computing where the data is" is taken one step further. In concrete approaches, the physical and/or electrical properties of the local memory are exploited to perform basic mathematical operations within the memory storage cells directly. In such a "process-in-memory" (PIM) concept, the programmable local, for example random access or content-addressable, memory may comprise a PIM unit, which may be programmed in a manner comparable to the programming of FPGAs.

For example, the programmable local memory may be a phase-change random access memory and/or a resistive random access memory. A phase-change random access memory (PRAM) may, for example, use chalcogenide glass as a medium capable of switching between a low- and a high-resistive phase. In-memory computing capabilities can be provided by exploiting Kirchhoff's circuit laws and analogue storage capabilities. In resistive content-addressable memory (ReCAM) the resistance within a memory cell (bit) is modulated by creating and repairing defects within an oxide layer.

Using such a programmable local memory, which allows programmed software means to run thereon, provides the option of allowing data selection processes, in particular searching and/or filtering, to be performed in the local memory itself while keeping the at least one required source file open and loaded during the whole analysis process. That is, analysis software components do not need to open the required source file every time data therefrom is needed, but simply send a source data request to the selector interface of the selector software, which invokes at least one respective selector component of the selector software and selects the requested source data from the continuously open and loaded source file, such that the selector software can provide it via the selector interface to the requesting analysis software component.

The analysis software component can then swiftly continue analysis in the analysis process, such that result information of the current analysis process can be obtained.

Advantageously, at least a part of the at least selector component may be programmed into the programmable local memory and executed by (and hence in) the programmable local memory. That is, at least a part of the at least one selector component of the selector software to provide requested source data may be executed directly inside the programmable local memory. In this manner, loading source data required to execute the selector component into the processor cache can be omitted. The results of the selector components executed in the programmable local memory are provided via the selector interface and processed by the analysis software component. Transfer of source data from the programmable local memory into the processor cache for executing selector components is no longer required.

In preferred embodiments, at least all selector components of the selector software may be executed in the local memory, completely obviating the need to transfer more than the requested source data into the processor cache.

Preferably, in addition to the at least one selector component, the selector software may comprise a central component providing the interface and/or handling the requests and/or controlling the at least one selector component. In particular, the central component may invoke selector components based on a source data request. The central component may, in particular, also be programmed into and executed in the programmable local memory, but is preferably executed in at least one of the at least one processor. The central component of the selector software can be started with the analysis process, determine the required source file or source files, transfer the at least one required source file from the long-time storage into the programmable local memory and provide the selector interface for the at least one analysis software component of the current analysis process.

Generally speaking, by the combination of a hardware means and a software means, namely the programmable local memory and the selector software, also the advantages may be expediently combined. The required source files, that is, the genomic files, only have to be transferred from the long-time storage into the local memory once. Hence, the total time required for transferring required source files for the complete data analysis of a case can be reduced to a minimum, since the single analysis steps performed by analysis software components do not have to repeat this transfer. On the other hand, by completely or partly executing selector components in the programmable local memory itself, the time for transferring source data required for executing the selector components from the programmable local memory into the processor cache may also be reduced to a minimum. Furthermore, executing selector components in the programmable local memory significantly reduces energy consumption for performing the analysis process. Providing the selector interface allows to implement the analysis software components on a more abstract level and to reduce the complexity.

In summary, this leads to a reduced time-to-solution for the whole data analysis pipeline and reduced working costs and complexity. This is particularly important for time sensitive processes, in particular regarding result information forming a basis for diagnosis. For example, the invention may be advantageously applied in cancer diagnosis, in particular leukemia diagnosis, where time is of the essence.

Here, generally speaking, the source data may in particular comprise a whole genome sequence and/or a whole exome and/or targeted panels of a genome sequence and/or an exome. It has been found that the structure of corresponding source files (genome files) is particularly suitable for selector components programmed into the programmable local memory. In particular, source data requests in this context often relate to patterns, which can be found or filtered advantageously by selector components running in the programmable local memory.

In a first step, in particular by the central component, at least one required source file for a current analysis process is determined. In particular, the at least one required source file may be determined from a parametrization of the current analysis process. Often, the analysis software for such analysis processes is already started giving required source files, in particular containing the source data to be analyzed, as a parameter, which can be read and further used by the selector software, in particular the central component, to identify the required source files. The at least one source file can then be loaded into the programmable local memory and held there for selection of requested source data by selector components.

Here, as already discussed above, source data describing at least one genome sequence usually has a large size. For example, whole genome sequences may have a size of 1 TB, exomes the size of several hundred GB and even targeted panels may still have sizes up to 100 GB. Hence, preferably, the local memory may comprise at least 100 GB, in particular up to several TB, and/or the at least one source file may have a size of at least 10 GB. In concrete embodiments, the at least one source file may be in the FASTQ format and/or in the BAM format and/or in the CRAM format and/or in the NIB format.

In embodiments, at least one source data request may comprise a requested pattern, wherein the at least one selector component searches and/or filters the source data regarding the pattern. In particular, the pattern may comprise a sequence of nucleotide bases. Usually, A, T, C, and G are used for the nucleotide bases. The at least one selector component may search and/or filter the genomic source data for these patterns and select, for example, source data comprising the pattern, following the pattern and/or preceding the pattern.

In some embodiments, the source data may be mapped regarding a reference sequence and at least one source data request may comprise at least one requested region, wherein searching and/or filtering is restricted to the at least one requested region. Hence, if the source data has already been mapped regarding a reference genome, regions have been defined which may also be addressed in source data requests. For example, filtered lists regarding the whole source data or at least one certain region may be selected as requested source data and provided via the selector interface.

As the storage, a local storage, in particular a hard disk, of the processing device may be used. Additionally and/or alternatively, a cloud storage may be used. For example, source files may already have been downloaded from a cloud storage to a local storage, in particular a hard disk, for example an SDD and/or an HDD. In other embodiments, the long-time storage may be one of a sequencing device. In particular, if a cloud storage is used as the storage, significant reduction of time-to-solution can be achieved, however, also regarding loading data from local storages into the programmable local memory, notable improvement is made.

In a preferred embodiment, access to the processing device may be provided in a cloud environment. For example, the processing device may be provided as a virtual computer, wherein, still, the processor and the programmable local memory are associated to each other in hardware. Just as dedicated solutions providing virtual processing devices having dedicated FPGAs have already been proposed, the invention allows to implement the processing device having the programmable local memory in a cloud, providing the basis for performing analysis processes.

A processing device for analyzing source data describing a genome sequence according to the invention has at least one processor, at least one local memory used by the at least one processor, and a communication interface to a storage, wherein the source data is stored in at least one source file. According to the invention, the local memory is a programmable local memory, which is configured to execute at least one software means, and the processing device further comprises a selector software, which is at least partly executed in the programmable local memory, wherein, to perform a current analysis process on at least one required source file, the selector software is configured to
- load the at least one required source file from the storage into the programmable local memory,
- provide at least one selector interface to at least one analysis software component, which is executed on at least one of the at least one processor, of the current analysis process, and,
- if a source data request from at least one of the at least one analysis software component is received via the selector interface, select requested source data from the at least one required source file in the programmable local memory by at least one selector component of the selector software according to the source data request and provide the selected source data via the selector interface to the requesting analysis software component.

All features and remarks regarding the method according to the invention analogously apply to the processing device according to the invention and vice versa, such that the same advantages can be achieved.

In particular, the selector software may comprise
- at least one central component for providing the selector interface and for determining and loading the at least one required source file, and
- the at least one selector component controllable by the central component for selecting the source data and providing the requested source data,
wherein at least a part of the at least one selector component is programmed into the programmable local memory and executable by the programmable local memory. In particular, all selector components may be completely programmed into the programmable local memory and executable by the programmable local memory. Preferably, the central component may be executed by the at least one processor.

As already discussed regarding the method, the programmable local memory preferably comprises at least 100 GB. Furthermore, the storage may be a local storage, in particular a hard disk, of the processing device and/or a cloud storage. As further already discussed above, the programmable local memory may be phase-change random access memory and/or a resistive content-addressable memory.

The invention also concerns a processing system, comprising a processing device according to the invention provided in a cloud environment. All features and remarks regarding the processing device and the method also apply to the processing system according to the invention. In particular, the processing device and the processing system may be configured to execute the steps of a method according to the invention.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: a functional drawing of a processing device according to the invention, and
- Fig. 2: a flow chart illustrating an embodiment of a method according to the invention.

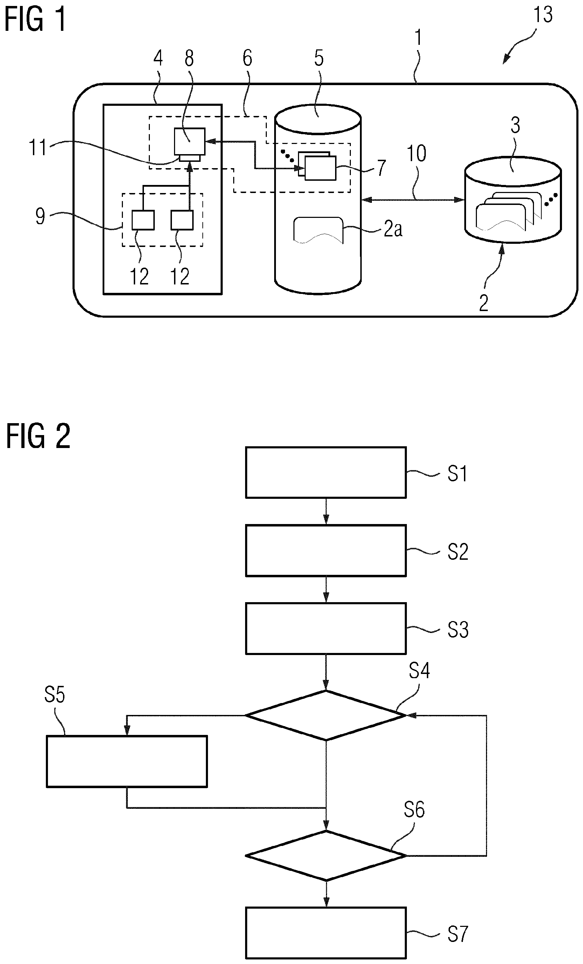

Fig. 1 is a principle, functional drawing of a processing device 1 according to the invention. The processing device 1 serves to analyse source data describing a genome sequence in at least one analysis process. The source data are stored in source files 2, which may also be called genomic files. The source files 2 may be in the FASTQ format, the BAM format, the CRAM format, the NIB format, or any other format used for genomic source data. The source files 2 have a size of at least 10 GB. If a whole exome is covered, the size may be several 100 GB, or, for whole genomes, at least 1 TB.

The source files 2 are stored in a non-volatile long-time storage 3, which may be a local storage of the processing device 1 and/or a cloud storage, which can be accessed by the processing device 1 via a communication interface. In the case of a local, internal storage 3, the communication interface is internal.

The processing device 1 further comprises at least one processor 4 using an associated local memory 5, in this case a local random access memory (RAM). In other embodiments, the local memory 5 may also be a content-addressable memory (CAM). The local memory 5 is programmable and may, for example, be a phase-change random access memory. A selector software 6 of the processing device 1 comprises selector components 7 which are programmed into the programmable local memory 5 and executable in the programmable local memory 5 and a central component 8.

If an analysis process regarding the source data, for example implemented by analysis software 9, is started in the processor 4, the central component 8 is also started in the processor 4. The central component 8 determines at least one required source file 2a of the source file 2, for example from a parametrization of the current analysis process. The central component 8 loads the respective required source file 2a via the communication interface into the programmable local memory 5 by transferring it via the communication link 10 between the storage 3 and the programmable local memory 5. The at least one required source file 2a is held in the programmable local memory 5 for as long as the current analysis process runs. To provide enough memory space, the programmable local memory 5 comprises at least 100 GB. It is further noted that the data line between the at least one processor 4 and the local programmable memory 5 has an at least tenfold higher data transfer rate compared to the communication link 10, i.e. has a significantly larger bandwidth.

The central component 8 provides a selector interface 11 to analysis software components 12 of the current analysis process. The central component 8 receives source data requests describing requested source data from the at least one required source file 2a. In response to such a source data request, the central component 8 runs at least one corresponding selector component 7 in the programmable local memory 5. The selector component 7 selects the requested source data according to the source data request from the at least one required source file 2a and the central component 8 provides the requested source data selected by the selector component 7 via the selector interface 11 to the requesting analysis software component 12. That is, only the requested source data has to be transferred into a processor cache of the processor 4.

Hence, the at least one required source file 2a only needs to be transferred once for all analysis steps and only requested source data actually has to be transferred into the processor cache of the at least one processor 4, since the selector component 7 is running in the programmable local memory 5 itself.

It is noted that providing such a standardized selector interface 11 by the selector software 6 also simplifies programming and reduces complexity of the analysis software 9. In particular, the selector software 6 can replace libraries used in the state of the art to implement the selection processes in analysis software components 12 themselves.

Hence, the processing device 1 provides an easy-to-use, time-optimized platform which can in particular also be provided as a processing system 13 in a cloud environment.

Fig. 2 is a flow chart of a method according to the invention as it can be executed in an embodiment of the processing device 1 according to the invention as described with respect to fig. 1. In a step S1, an analysis process is started, in particular by starting the analysis software 9 on the at least one processor 4 using parameters indicating which source data is to be analyzed, in particular the at least one required source file 2a. In a step S2, the central component 8 is started and determines the at least one required source file 2a from the parametrization. In a step S3, the at least one required source file 2a is transferred from the storage 3 into the programmable local memory 5 and the selector interface 11 is initialized.

In a step S4, source data requests are awaited by the central component 8 from analysis software components 12. For example, the source data requests may comprise a requested pattern, in particular a sequence of nucleotide bases. In such a case, the selector components 7 are configured to search and/or filter the source data in the at least one required source file 2a regarding the pattern. If the source data is mapped regarding a reference sequence, source data requests may also comprise at least one requested region, such that searching and/or filtering can be restricted to the at least one requested region.

If a source data request is received from an analysis software component 12, in a step S5, at least one corresponding selector component 7 is run by the central component 8 in the programmable local memory 5 to select the requested source data from the source data of the at least one required source file 2a in the programmable local memory 5. For example, sequence sections or segments comprising the pattern may be returned as requested source data. These requested source data are transferred to a processor cache of the at least one processor 4 and can then be further processed by the analysis software component 12.

In a step S6, it is checked whether the current analysis process is completed. If this is not the case, it is returned to step S4. If further source data requests are received, requested source data can be swiftly provided since the at least one required source file 2a is still held in the programmable local memory 5 and the selector component 7 is run directly in the programmable local memory 5.

If the current analysis process is completed, result information is provided in a step S7. The result information may be further evaluated, for example to support a physician regarding a cancer diagnosis, in particular a leukaemia diagnosis. By combining the advantages of the programmable local memory 5 and of the selector software 6, which is at least partly implemented in the programmable local memory 5, the time-to-solution can be significantly reduced.

It is noted at this point that, of course, the selector software 6, in particular its parts programmed into the programmable local memory 5, can also be updated since the programming of the programmable local memory 5 can be adapted or changed. For example, such an update may be compared to a firmware update and/or re-programming of an FPGA.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

### Reference numeral list

- 1: processing device
- 2, 2a: source file
- 3: storage
- 4: processor
- 5: local memory
- 6: selector software
- 7: selector component
- 8: central component
- 9: analysis software
- 10: communication link
- 11: selector interface
- 12: analysis software component
- 13: processing system

- S1 - S7: step

## Claims

1. Computer-implemented method for analyzing source data describing a genome sequence in a processing device (1) having at least one processor (4) and at least one local memory (5) used by the at least one processor (4), wherein the source data is stored in at least one source file (2, 2a) in a storage (3),
**characterized in that** a programmable local memory (5), which is configured to execute at least one software means, and a selector software (6), which is at least partly executed in the programmable local memory (5), are used, wherein
- at least one required source file (2a) for a current analysis process is determined,
- the at least one required source file (2a) is loaded by the selector software (6) from the storage (3) into the programmable local memory (5),
- the selector software (6) provides at least one selector interface (11) to at least one analysis software component (12), which is executed on at least one of the at least one processor (4), of the current analysis process,
- wherein, if a source data request from at least one of the at least one analysis software component (12) is received via the selector interface (11), requested source data is selected from the at least one required source file (2a) in the programmable local memory (5) by at least one selector component (7) of the selector software (6) according to the source data request and provided via the selector interface (11) to the requesting analysis software component (12).

2. Method according to claim 1, **characterized in that** at least a part of the at least one selector component (7) is programmed into the programmable local memory (5) and executed by the programmable local memory (5).

3. Method according to one of the preceding claims, **characterized in that** the selector software (6) comprises a central component (8) providing the selector interface (11) and/or handling the source data requests and/or controlling the at least one selector component (7).

4. Method according to one of the preceding claims, **characterized in that** the programmable local memory (5) comprises at least 100 GB and/or the at least one source file (2, 2a) has a size of at least 10 GB and/or that the at least one source file (2, 2a) is in the FASTQ format and/or in the BAM format and/or in the CRAM format and/or in the NIB format.

5. Method according to one of the preceding claims, **characterized in that** at least one source data request comprises a requested pattern, wherein the at least one selector component (7) searches and/or filters the source data regarding the pattern.

6. Method according to claim 5, **characterized in that** the source data is mapped regarding a reference sequence and at least one source data request comprises at least one requested region, wherein searching and/or filtering is restricted to the at least one requested region.

7. Method according to claim 5 or 6, **characterized in that** the pattern comprises a sequence of nucleotide bases.

8. Method according to one of the preceding claims, **characterized in that**, as the storage (3), a local storage, in particular a hard disk, of the processing device (1), and/or a cloud storage is used.

9. Method according to one of the preceding claims, **characterized in that** access to the processing device (1) is provided in a cloud environment.

10. Processing device (1) for analyzing source data describing a genome sequence, the processing device (1) having at least one processor (4), at least one local memory (5) used by the at least one processor (4), and a communication interface to a storage (3), wherein the source data is stored in at least one source file (2, 2a), **characterized in that** the local memory (5) is a programmable local memory (5), which is configured to execute at least one software means, and the processing device (1) further comprises a selector software (6), which is at least partly executed in the programmable local memory (5), wherein, to perform a current analysis process on at least one required source file (2a), the selector software (6) is configured to
- load the at least one required source file (2a) from the storage (3) into the programmable local memory (5),
- provide at least one selector interface (11) to at least one analysis software component (12), which is executed on at least one of the at least one processor (4), of the current analysis process, and,
- if a source data request from at least one of the at least one analysis software component (12) is received via the selector interface (11), select requested source data from the at least one required source file (2a) in the local memory (5) by at least one selector component (7) of the selector software (6) according to the source data request and provide the selected source data via the selector interface (11) to the requesting analysis software component (12) .

11. Processing device (1) according to claim 10, **characterized in that** the selector software (6) comprises at least one central component (8) for providing the selector interface (11) and for loading the at least one required source file (2a) and the at least one selector component (7) controllable by the central component (8) for selecting the source data and providing the requested source data, wherein at least a part of the at least one selector component (7) is programmed into the programmable local memory (5) and executable by the programmable local memory (5).

12. Processing device (1) according to one of claims 10 and 11, **characterized in that** the programmable local memory (5) comprises at least 100 GB.

13. Processing device (1) according to one of claims 10 to 12, **characterized in that** the storage (3) is a local storage, in particular a hard disk, of the processing device (1) and/or a cloud storage.

14. Processing device (1) according to one of claims 10 to 13, **characterized in that** the programmable local memory (5) is a phase-change random access memory and/or a resistive content-addressable memory.

15. Processing system (13), comprising a processing device (1) according to one of claims 10 to 14 provided in a cloud environment.
